# EUROPÄISCHE PATENTANMELDUNG

(11) **EP 2 366 353 A1**
(43) Veröffentlichungstag der Anmeldung: **21.09.2011**
(21) Anmeldenummer: 10168259.9
(22) Anmeldetag: 02.07.2010
(51) Int. Cl.: A61B 18/14

(54) **Elektrochirurgische bipolare Schere für Koagulation und zum Sezieren**

(30) Priorität: 15.03.2010 LV 100033
(71) Anmelder: OOO "Novye Energetischeskie, Moskau 107045 (RU); "Golsen Limited", 3041 Limassol (CY)
(72) Erfinder: Belov, Sergey V., 119311 Moscow (RU); Borik, Mikhail A., 105264 Moscow (RU); Ganin, Igor Y., 124498 Zelenograd (RU); Danileyko, Yuri K., 142092 Troitsk (RU); Ivanov, Aleksandr D., 109378 Moscow (RU); Lomonova, Elena E., 129010 Moscow (RU); Nefedoc, Sergey M., 115612 Moscow (RU); Osiko, Vjacheslav V., 117420 Moscow (RU); Salyuk, Viktor A., 117321 Moscow (RU)
(74) Vertreter: Jeck, Anton

(57) **Zusammenfassung**

Die Erfindung betrifft eine elektrochirurgische bipolare Schere für Koagulation und zum Sezieren, die aus zwei isolierten Branchen besteht, wobei die Branchen in Bezug aufeinander mittels eines elektrisch isolierten Gelenks beweglich verbunden sind und an dem proximalen Ende Ringe für die Finger sowie eine Anschlussstelle für elektrischen Strom aufweisen und wobei das distale Ende der Branchen mit Schneidekanten versehen ist. Damit die Schere bei einer Durchtrennung die Koagulation der Biogewebe mit gleicher Tiefe der Durchtrennebene sicherstellt und eine erhöhte Schneidfähigkeit und Verschleißfähigkeit aufweist, sieht die Erfindung vor, dass die beiden distalen Enden der Schere eine Verbundstruktur aus Metall und Dielektrikum aufweisen.

## Beschreibung

Die Erfindung betrifft eine elektrochirurgische Schere für Koagulation nach dem Oberbegriff des Anspruchs 1.

Die Erfindung ist als medizinisches elektrochirurgisches Instrument einsetzbar. Sie kann bei der Durchführung von chirurgischen Eingriffen zur gleichzeitigen Trennung und Koagulation der biologischen Gewebe, zum Abbinden der Blutgefäße in offenen Operationsfeldern und bei laparoskopischen Operationen angewendet werden. Die bipolare Schere ermöglicht eine Durchtrennung der Gewebe mit einer gleichzeitigen Koagulation. Sie dient auch zur bipolaren Koagulation einzelner Gewebebereiche. Eine Blutstillung erfolgt durch eine kontrollierbare Erwärmung der Biogewebe mittels elektrischer HF-Ströme. Bei der bipolaren Schere sind beide Elektroden aktiv und in einem Instrument miteinander verbunden.

Aus der RU 5675103 ist eine elektrochirurgische bipolare Schere bekannt. Diese elektrochirurgische Schere besteht aus gekreuzten isolierten Branchen. Die Branchen sind aus einem stromleitenden Material ausgebildet, die mittels eines elektrisch isolierten Gelenks gekoppelt sind. Der Mangel dieser bipolaren elektrochirurgischen Schere ist eine Mitwirkung der Metallelektroden beim Koagulationsvorgang. Die Teile dieser Elektroden sind gleichzeitig die Schneideklingen. Trotz einer spezifischen Behandlung und Härtung der Scherbacken aus Metall, die unmittelbar mit dem Biogewebe zusammenwirken, kommt eine schnelle Degradation der Scherbacken bei ihrem Einsatz infolge der elektrochemischen Abläufe zustande. Diese Degradation führt zu einer Verschlechterung der Schneidefähigkeit der Schere. Dabei kann eine ungewünschte Verletzung der durchtrennten Biogewebe vorkommen.

Der nächst kommende Stand der Technik gegenüber der vorgeschlagenen Erfindung ist eine elektrochirurgische bipolare Schere gemäß dem US 5324289 "Hemostatic bipolar electrosurgical cutting and methods of use". Diese Schere ist als zwei gekreuzte Branchen ausgebildet, die mittels einer abisolierten Achse mit Schneidekanten an distalen Klingen verbunden sind. An proximalen Enden der Branchen sind Ringe für die Finger sowie Elektroden zum Anlegen eines elektrischen Stroms vorgesehen. Eine Schneidekante der Branche ist mit einer keramischen Aufdampfschicht versehen. Die zweite Schneidekante ist aus medizinischem Stahl ausgebildet. Die Mängel dieser Einrichtung sind Folgende:
1. eine unmittelbare Mitwirkung der Metallelektrode - der Schneide beim Sezieren und bei der elektrischen Koagulation und folglich eine elektrochemische Zerstörung der Elektrode;
2. eine unsymmetrische Koagulation an beiden Seiten der Durchtrennebene.

Es ist Aufgabe der Erfindung, eine elektrochirurgische Schere zu entwickeln, welche bei einer Durchtrennung die Koagulation der Biogewebe mit gleicher Tiefe der Durchtrennebene sicherstellt. Dabei muss diese Schere eine erhöhte Schneidefähigkeit und Verschleißfestigkeit sicherstellen.

Die gestellte Aufgabe wird durch die Merkmale des Anspruchs 1 gelöst.

Weitere zweckmäßige Ausgestaltungen der Erfindung gehen aus den Unteransprüchen hervor.

Die elektrochirurgische bipolare Schere weist zwei gekreuzte isolierte Branchen 2 auf, die aus einem stromleitenden Material ausgebildet sind. Die Branchen sind beweglich mittels eines elektrisch isolierten Gelenks 3 verbunden. An den proximalen Enden der Branchen 2 sind zwei Ringe für die Finger sowie Anschlussstellen 1 zum Anlegen eines elektrischen Stroms vorgesehen. Die Schneidekanten der Klingen sind durch dielektrische Teile 5 von zweilagigen Verbundstoffstrukturen ausgebildet. Sie sind am distalen Ende 4 der Branchen 2 angeordnet. Die Außenschicht der distalen Enden 4 der Branchen 2 ist aus medizinischem Stahl gefertigt.

Bei laparoskopischen Operationen besteht die elektrochirurgische bipolare Schere aus einem Arbeitsteil. Der Arbeitsteil weist an seinem distalen Ende 4 zwei stromleitende isolierte Klingen auf. Innerhalb eines Verbindungsrohrs 7 ist eine Zugstange angeordnet. Diese beginnt an den Ringen des Griffhebels 2 und endet an einer Vorrichtung 8, welche die Klingen der Schere öffnet und schließt. Die Klingen weisen verdünnte und abgerundete Enden auf. Die Ringe für die Finger und die Anschlussstellen 1 zum Anlegen des elektrischen Stroms sind am Griffhebel 2 vorgesehen. Die Schneidekanten der Klingen sind durch dielektrische Teile 5 von zweilagigen Verbundstoffstrukturen ausgebildet, welche am distalen Ende 4 der Branchen 2 angeordnet sind. Die Außenschicht der distalen Enden 4 der Branchen 2 ist aus medizinischem Stahl gefertigt. Die Griffe der Schere sind mit Isolierteflonbeschichtung versehen.

Die Erfindung wird anhand eines in den Zeichnungen dargestellten Ausführungsbeispiels näher erläutert.

Es zeigen:

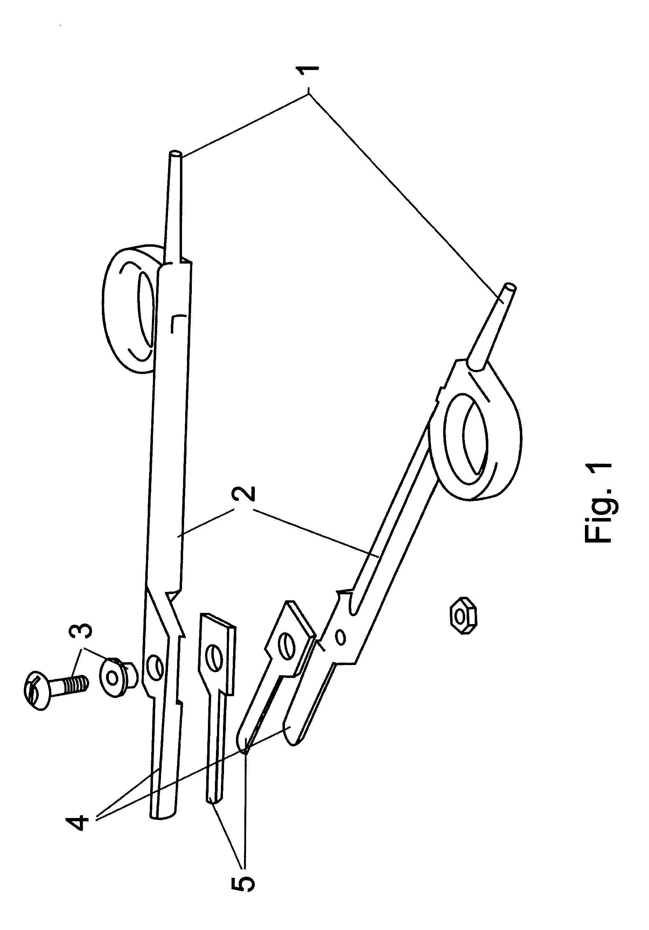

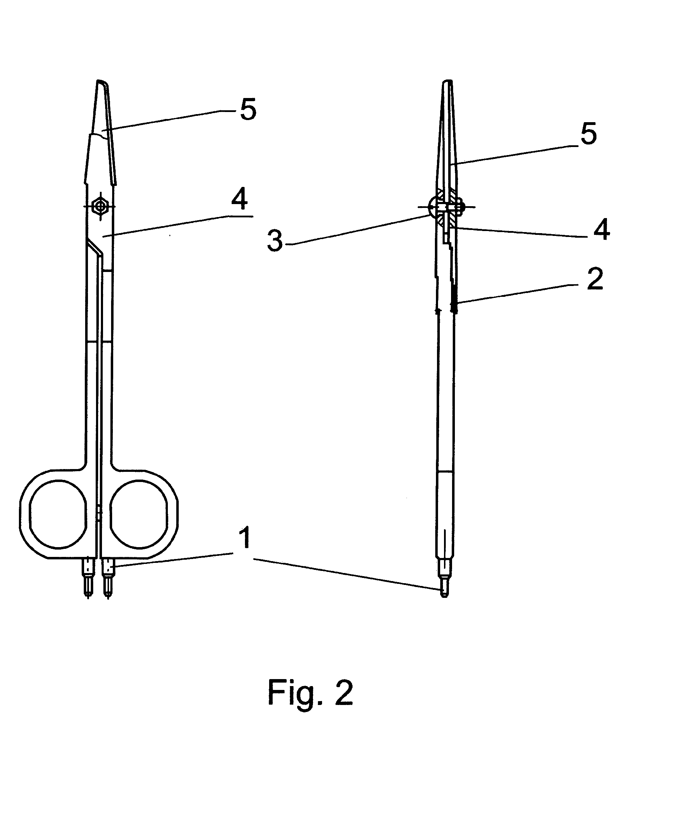

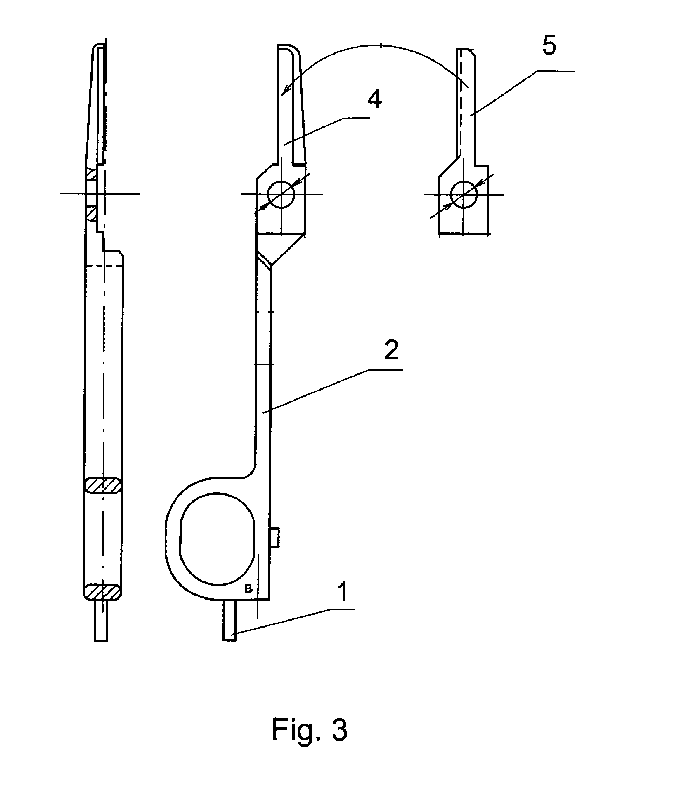

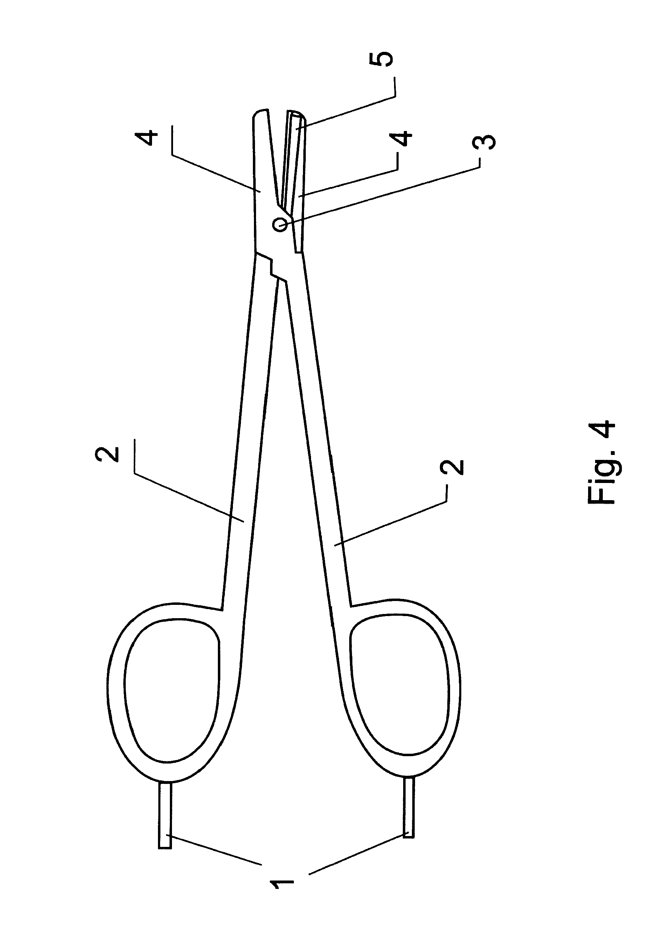

- Fig. 1 - 4: die Schere gemäß der Erfindung,
- Fig. 5: eine Schere für laparoskopische Operationen,

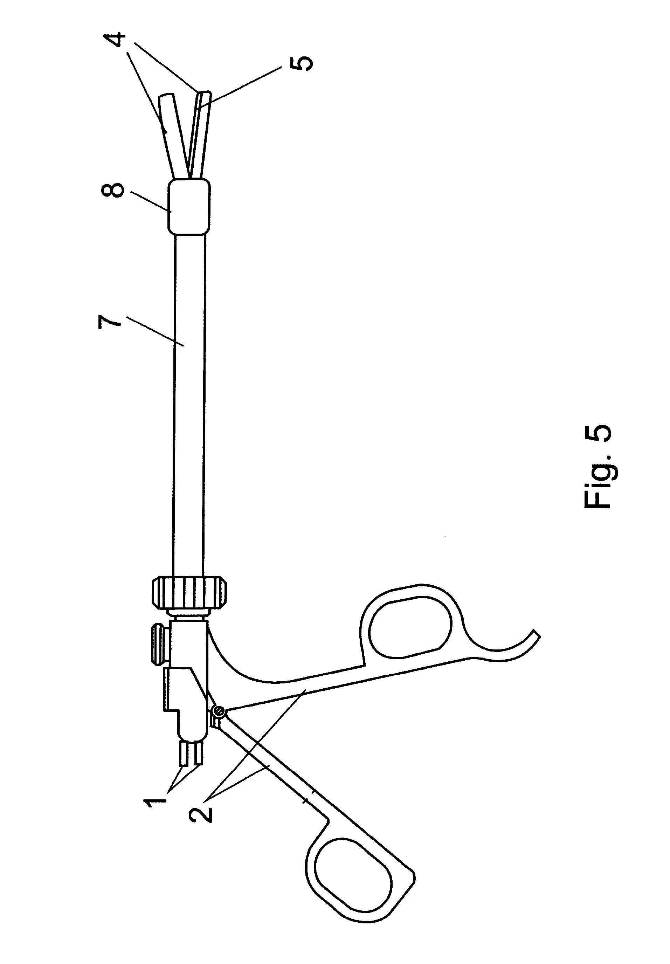

- Fig. 5: die Funktionsweise der Schere während der Durchtrennung und der Koagulation und

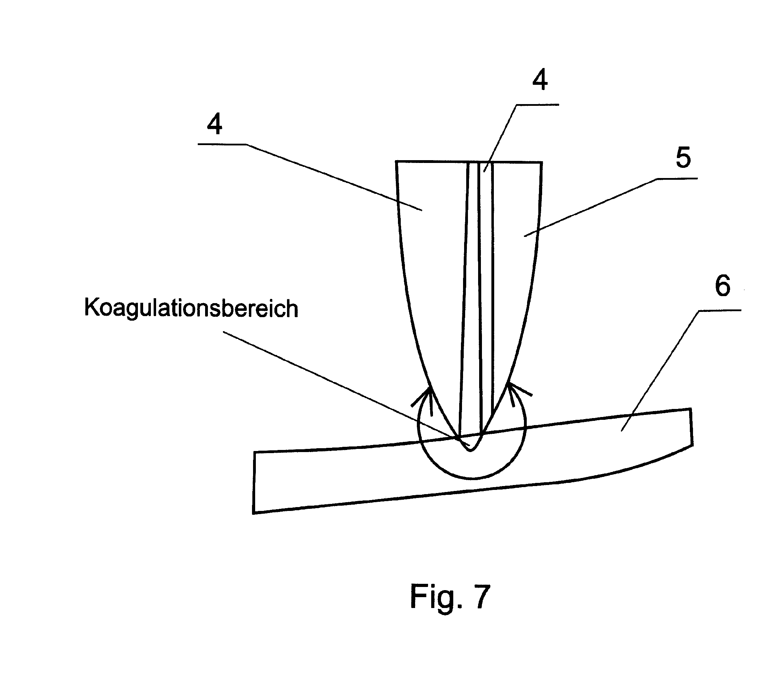

- Fig. 7: ein Anwendungsbeispiel der Schere für eine präzise punktförmige Koagulation einer lokalisierten Blutung.

Die Stärke des dielektrischen Teils der zweilagigen Verbundstoffstruktur 5 ist einerseits durch eine maximale für die Koagulation benötigte Stromdichte (und zwar ansteigend) begrenzt. Andererseits kann die Minderung der Stärke des dielektrischen Teils die Zerstörung der Metallelektroden wegen der elektrochemischen Abläufe verursachen. Versuche haben gezeigt, dass die Optimalstärke des dielektrischen Teils der Klingen innerhalb von 0,2 - 2,0 mm liegen muss.

Als Dielektrikum wird in der zweilagigen Verbundstoffstruktur der Klingen ein kristallines nanostrukturiertes, teilweise stabilisiertes Zirkondioxid verwendet. Dabei kann der erwähnte dielektrische Teil der zweilagigen Verbundstoffstruktur der Branchen 2 sowohl als auswechselbarer Einsatz als auch als unlösbare Verbindung mit dem Metallteil des distalen Endes 4 der Branchen 2 ausgebildet sein. Die dielektrische Klinge kann eine variable Stärke aufweisen, die im Bereich von 0,2 bis 2,0 mm, wie oben angegeben, liegt.

Die Funktionsweise der vorgeschlagenen elektrochirurgischen bipolaren Schere ist wie folgt:

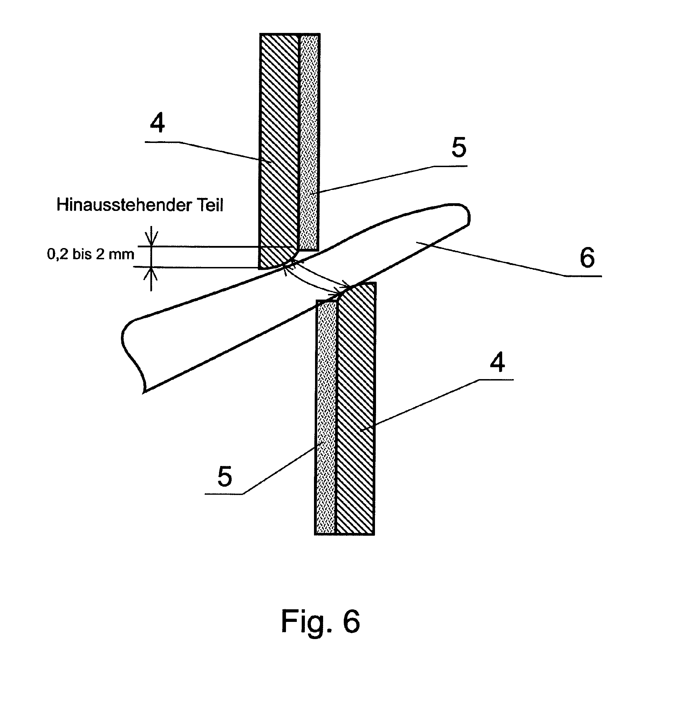

Ein HF-Strom wird über ein Kabel von einem Stromerzeuger an die Elektroden zur Stromeinspeisung 1 angelegt. Die Elektroden sind von der Konstruktion her mit den distalen Enden 4 der Scherenbranchen 2 vereinigt. Dabei stellen die voneinander abisolierten distalen Enden 4 der Branchen 2 eines solchen Instruments aktive bipolare Elektroden dar. Die Arbeitselemente der Schere (die distalen Enden 4) haben ständig zwei Kontaktpunkte mit dem Biogewebe. Der HF-Strom wird an das Instrument angelegt und fließt zwischen zwei Kontaktpunkten der Elektroden mit dem biologischen Gewebe 6. Dabei passiert dieser Strom über den kürzesten Weg und zwar nur über jenen Gewebebereich, der zwischen diesen zwei Punkten liegt. Somit wird die Koagulation ausgelöst (in Fig. 6 mit Pfeilen abgebildet). Der Strom wird zwischen zwei aktiven Elektroden innerhalb eines kleinen Gewebebereichs des Patienten lokalisiert. Dabei ist der Körper des Patienten kein Bestandteil des Stromkreises. Das minimiert einen unkontrollierbaren Wärmeschaden anderer Gewebe. Beim Schließen der Branchen 2 wird das biologische Gewebe 6 zwischen den distalen Enden 4 der Branchen 2 zusammengedrückt. Die dielektrischen Teile 5 der Klinge trennen mechanisch das biologische Gewebe. Das heißt, die Durchtrennung des biologischen Gewebes 6 erfolgt durch die Außenkanten der dielektrischen Teile der zweilagigen Verbundstoffstruktur des distalen Endes 4 der Branchen 2. Die Metallteile 4 der zweilagigen Branchen 2 aus Verbundstoff stehen über die Schneidekante der dielektrischen Teile 5 hinaus. Bei der Durchtrennung des biologischen Gewebes 6 stellen diese Metallteile 4 die Elektrokoagulation des Biogewebes dar und zwar mit einer räumlichen Überholung in Bezug auf den Durchtrennungsvorgang. Dabei muss die Tiefe der Elektrokoagulation der Durchtrennebene überall gleich sein. Der dielektrische Teil der zweilagigen Verbundstoffstruktur der Branchen 2 kann in Form von auswechselbaren Einsätzen ausgebildet sein. Das erlaubt es, die Höhe des hinaus stehenden Metallteils 4 über der Schneidekante der dielektrischen Teile 5 der zweilagigen Branchen 2 aus Verbundstoff zu ändern. Dies ermöglicht es, die Größe der räumlichen Überholung des Koagulationsvorgangs, verglichen mit dem Durchtrennungsvorgang, zu ändern. Die Höhe des hinaus stehenden Metallteils 4 über der Schneidekante 5 liegt im Bereich von 0,2 bis 2 mm, je nach der Struktur des Biogewebes. Eine solche Ausführung der bipolaren Schere ermöglicht es, die Durchtrennung der Gewebe mit gleichzeitiger Koagulation bei der Durchführung von chirurgischen Eingriffen in offenen Operationsfeldern und bei laparoskopischen Operationen vorzunehmen sowie eine bipolare Koagulation einzelner Gewebebereiche zu erzielen. In Fig. 7 ist ein Beispiel für den Einsatz der Schere für eine punktförmige Koagulation der lokalisierten Blutung dargestellt. Dadurch entfällt die Anwendung von zusätzlichen Instrumenten. Folglich wird auch die Operationsdauer vermindert.

## Patentansprüche

1. Elektrochirurgische bipolare Schere für Koagulation und zum Sezieren, die aus zwei isolierten stromleitenden Branchen (2) besteht, wobei die Branchen (2) in Bezug aufeinander mittels eines elektrisch isolierten Gelenks (3) beweglich verbunden sind und an dem proximalen Ende Ringe für die Finger sowie eine Anschlussstelle (1) für elektrischen Strom aufweisen und wobei das distale Ende (4) der Branchen (2) mit Schneidekanten (5) versehen ist,
**dadurch gekennzeichnet,**
**dass** die beiden distalen Enden (4) der Schere eine Verbundstoffstruktur (4, 5) von Metall und Dielektrikum aufweisen.

2. Elektrochirurgische bipolare Schere nach Anspruch 1,
**dadurch gekennzeichnet,**
**dass** die Schneidekanten der Klingen als dielektrische Teile (5) einer zweilagigen Verbundstoffstruktur (4, 5) ausgebildet sind.

3. Elektrochirurgische bipolare Schere nach Anspruch 1,
**dadurch gekennzeichnet,**
**dass** in der Verbundstoffstruktur (4, 5) der Klingen ein kristallines nanostrukturiertes, teilweise stabilisiertes Zirkondioxid als Dielektrikum (5) eingesetzt ist.

4. Elektrochirurgische bipolare Schere nach Anspruch 2,
**dadurch gekennzeichnet,**
**dass** der dielektrische Teil (5) der zweilagigen Verbundstoffstruktur (4, 5) der Branchen (2) sowohl als auswechselbarer Einsatz als auch in Form einer unlösbaren Verbindung mit dem Metallteil (4) der distalen Enden (4) der Branchen (2) ausgebildet ist.

5. Elektrochirurgische bipolare Schere nach Anspruch 4,
**dadurch gekennzeichnet,**
**dass** der dielektrische Teil (5) der Klinge eine variable Stärke aufweist.

6. Elektrochirurgische bipolare Schere nach Anspruch 5,
**dadurch gekennzeichnet,**
**dass** die Stärke des dielektrischen Teils (5) der Klinge 0,2 mm bis 2 mm beträgt.

7. Elektrochirurgische bipolare Schere nach Anspruch 1,
**dadurch gekennzeichnet,**
**dass** die Durchtrennung eines Biogewebes (6) mittels der Außenkante des dielektrischen Teils (5) der zweilagigen Verbundstoffstruktur (4, 5) der Branchen (2) erfolgt.

8. Elektrochirurgische bipolare Schere nach Anspruch 1,
**dadurch gekennzeichnet,**
**dass** die Metallteile (4) der zweilagigen Branchen (2) aus Verbundstoff (4, 5) über die Schneidekante der Klingen um 0,2 mm bis 2 mm hinausragen.

9. Elektrochirurgische bipolare Schere nach Anspruch 8,
**dadurch gekennzeichnet,**
**dass** die hinaus stehenden Metallteile (4) der zweilagigen Branchen (2) aus Verbundstoff (4, 5) während der Durchtrennung von Biogewebe (6) eine Elektrokoagulation des Biogewebes (6) mit Überholung im Bezug auf den Durchtrennungsvorgang sicherstellen.

10. Elektrochirurgische bipolare Schere nach Anspruch 1,
**dadurch gekennzeichnet,**
**dass** die zweilagigen Branchen (2) aus Verbundstoff (4, 5) während der Durchtrennung von Biogewebe (6) eine Elektrokoagulation des Biogewebes (6) mit gleicher Tiefe von der Durchtrennebene sicherstellen.

11. Elektrochirurgische bipolare Schere nach Anspruch 1,
**dadurch gekennzeichnet,**
**dass** diese Schere eine punktförmige Koagulation einer lokalisierten Blutung ermöglicht.
